# FASCICULE DE BREVET EUROPEEN

(11) **EP 0 452 219 B1**
(45) Date de publication et mention de la délivrance du brevet: **15.06.1994**
(21) Numéro de dépôt: 91400966.7
(22) Date de dépôt: 10.04.1991
(51) Int. Cl.: A61M 25/10, A61F 2/06

(54) **Cathéter à endoprothèse**
Endoprothese-Katheter
Endoprosthesis catheter

(30) Priorité: 13.04.1990 FR 9004840
(43) Date de publication de la demande: 16.10.1991
(73) Titulaire: Boussignac, Georges, F-92160 Antony (FR); Labrune, Jean-Claude, F-92160 Antony (FR)
(72) Inventeur: Boussignac, Georges, F-92160 Antony (FR); Labrune, Jean-Claude, F-92160 Antony (FR)
(74) Mandataire: Bonnetat, Christian

(56) Documents cités:
- EP-A- 0 275 230
- WO-A-88/05671
- FR-A- 2 617 721
- US-A- 4 681 564

## Description

La présente invention concerne un cathéter pour conduit morphologique susceptible de mettre en place une endoprothèse, temporaire ou permanente, à l'intérieur dudit conduit morphologique.

Plus particulièrement, quoique non exclusivement, le cathéter conforme à la présente invention est destiné à conclure une intervention réalisée avec une sonde à dilatation ou un rayonnement laser dans un conduit morphologique au moins partiellement obturé par un athérome. L'invention sera plus particulièrement décrite ci-après en rapport avec cette application, ce qui toutefois ne doit pas être considéré comme une limitation de l'invention.

On sait que, pour désobturer un tel conduit morphologique, on introduit dans le conduit morphologique, jusqu'au niveau dudit athérome, l'extrémité distale, soit d'une sonde à dilatation susceptible d'expanser radialement ledit athérome, soit d'un cathéter à fibre optique susceptible de conduire un rayonnement laser apte à éliminer ledit athérome.

On a remarqué que, à la suite d'une telle expansion radiale ou d'une telle élimination par rayonnement laser, la paroi du conduit morphologique présente, à l'emplacement de l'athérome éliminé, de nombreuses aspérités ou membranes propices à la formation d'un nouvel athérome.

La présente invention a notamment pour objet de remédier à cet inconvénient.

A cette fin, selon l'invention, le cathéter pour conduit morphologique comportant un ballon gonflable, disposé à l'extrémité distale dudit cathéter et relié à l'extrémité proximale dudit cathéter par au moins un conduit de gonflage susceptible d'être relié à un dispositif de gonflage, ledit ballon gonflable pouvant prendre, soit un état dégonflé pour lequel il est plaqué contre ladite extrémité distale dudit cathéter, soit un état gonflé pour lequel il est plaqué par sa paroi externe contre la paroi interne dudit conduit morphologique, est caractérisé en ce que ledit ballon gonflable présente la forme d'un manchon entourant ladite extrémité distale dudit cathéter et relié à celle-ci par un raccord souple, lui-même relié audit conduit de gonflage et en ce que, à l'état gonflé, ledit manchon ménage un espace annulaire entre sa paroi interne et ladite extrémité distale dudit cathéter.

Ainsi, ledit manchon peut, à l'état dégonflé, être introduit sans difficulté à l'intérieur dudit conduit morphologique, au niveau d'un athérome éliminé, par l'intermédiaire dudit cathéter, puis, une fois mis en place, être gonflé pour constituer in situ une prothèse à section annulaire susceptible de recouvrir, et donc d'inhiber, les aspérités et autres éléments susceptibles d'initialiser la formation d'un nouvel athérome.

On remarquera que, éventuellement, le manchon gonflable peut être utilisé également comme moyen d'expansion radiale dudit conduit morphologique, à la place des ballons gonflables dont sont généralement pourvues les sondes à dilatation connues, et qu'en toutes circonstances --qu'il y ait ou non athérome-- le manchon du cathéter conforme à l'invention constitue un renfort local dudit conduit morphologique.

Une telle sonde connue à ballon gonflable, qui correspond à la partie précaractérisante de la revendication 1, est par exemple décrite par le document US-A-4 681 564. Dans ce document, plusieurs formes de ballons sont données à titre d'exemples. Cependant, aucune d'entre elles ne concerne un manchon susceptible de constituer une prothèse in situ, notamment du fait que toutes les formes de ballons décrites obturent le conduit morphologique dans lequel elles sont introduites.

Par ailleurs, le document WO-88/05671 décrit un dispositif médical pouvant être inséré dans un conduit morphologique pour isoler la paroi de celui-ci du liquide qui y circule. Ce dispositif comporte un manchon, qui ne présente pas la structure d'un ballon gonflable, mais qui est associé à au moins un ballon de gonflage auxiliaire, pouvant éventuellement être retiré dudit conduit morphologique après avoir plaqué ledit manchon contre la paroi de celui-ci.

Dans le cathéter de l'invention, afin de bien serrer ledit manchon gonflable contre l'extrémité distale dudit cathéter, lorsque ledit manchon est dégonflé, on prévoit de préférence une membrane élastique entourant ledit manchon. Ainsi, ledit manchon est comprimé radialement en direction du cathéter, ce qui facilite l'introduction de celui-ci dans le conduit morphologique. L'expansion radiale du manchon s'effectue alors à l'encontre de l'action élastique de ladite membrane.

Une telle membrane est réalisée en une matière implantable biocompatible, telle que le silicone ou le latex. Avantageusement, comme on le verra par la suite, cette membrane est ajourée et peut même présenter la forme d'un filet, afin de ne pas s'opposer à la circulation sanguine périphérique, lorsque le manchon est lui-même ajouré et est disposé à l'intersection de conduits morphologiques.

Par ailleurs, si le manchon est destiné à former une prothèse temporaire qui ne garde sa forme expansée radialement que sous l'action de la pression exercée par un fluide introduit dans le conduit de gonflage par ledit dispositif de gonflage, on voit que, lorsque l'on supprime l'admission du fluide de gonflage, ladite membrane élastique ramène le manchon dégonflé en position serrée autour de l'extrémité distale du cathéter, ce qui permet l'extraction du cathéter et de son manchon hors dudit conduit morphologique.

Dans un mode avantageux de réalisation, ledit conduit de gonflage est incorporé audit cathéter et est relié audit manchon par un raccord souple. Ainsi, ledit manchon est rendu solidaire dudit cathéter par ledit raccord.

Dans le cas où l'on désire obtenir une prothèse permanente à l'intérieur dudit conduit morphologique, on adresse audit manchon un fluide de gonflage susceptible de rigidifier ledit manchon dans son état gonflé et on fait en sorte que ledit raccord souple soit susceptible d'être rompu. Ainsi, après durcissement dudit fluide de gonflage, qui donne alors au manchon une forme rigide définitive, on peut sortir le cathéter du conduit morphologique en y laissant ledit manchon gonflé.

De préférence, en plus dudit conduit de gonflage, on prévoit un conduit de vidange du fluide de gonflage reliant ledit manchon à l'extrémité proximale du cathéter. Ainsi, il est possible de contrôler parfaitement le contenu du manchon en fluide et de réaliser par une vidange complète l'évacuation des éventuelles impuretés ou gaz pouvant exister dans le manchon. Il est alors avantageux que ce conduit de vidange, tout comme le conduit de gonflage, soit incorporé audit cathéter et soit relié audit manchon par un raccord souple. En cas de prothèse permanente, ce dernier raccord souple est également susceptible d'être rompu.

Avantageusement, le manchon est formé par un tube enroulé en serpentin hélicoïdal ou par un tube à double paroi. Afin de permettre la circulation homogène à travers la paroi du manchon, on prévoit que lesdites spires ne sont pas jointives ou on ménage des ouvertures dans le tube à double paroi.

Les figures du dessin annexé feront bien comprendre comment l'invention peut être réalisée. Sur ces figures, des références identiques désignent des éléments semblables.

La figure 1 est une coupe longitudinale schématique illustrant la mise en place du manchon dégonflé à l'intérieur d'un conduit morphologique.

La figure 2 est une coupe transversale selon la ligne II-II de la figure 1.

La figure 3 est une coupe longitudinale schématique illustrant la mise en place du manchon gonflé à l'intérieur du conduit morphologique.

La figure 4 est une coupe transversale selon la ligne IV-IV de la figure 3.

La figure 5 montre en perspective agrandie un mode de réalisation du manchon, sous la forme d'un serpentin.

La figure 6 montre, également en perspective agrandie, une variante de réalisation du manchon selon l'invention.

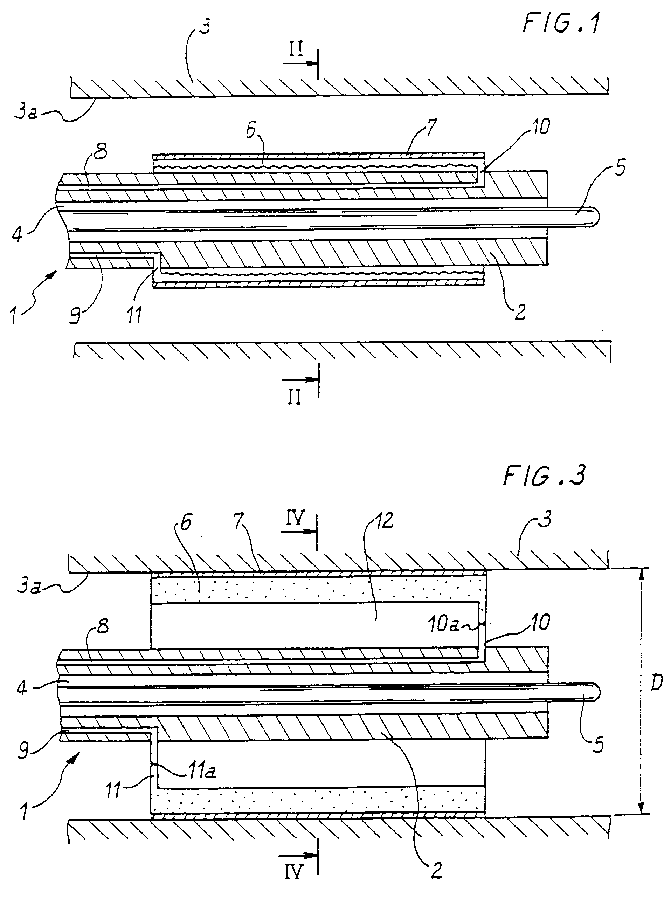

Le cathéter 1, dont l'extrémité distale 2 est représentée sur les figures 1 à 4, est introduit dans un conduit morphologique 3, par exemple une artère. Ce cathéter 1, qui est représenté sous la forme d'un tube, est percé d'un passage longitudinal 4 dans lequel est disposé un support de guidage 5 destiné à introduire ledit cathéter 1 à l'intérieur du conduit morphologique 3 selon une technique bien connue.

L'extrémité distale 2 du cathéter 1 est entourée par un manchon gonflable 6, lui-même comprimé en direction de ladite extrémité distale 2 par une membrane élastique 7.

Dans la paroi du cathéter 1 sont prévus au moins deux conduits 8 et 9, respectivement reliés audit manchon gonflable 6 par des raccords souples 10 et 11. L'extrémité proximale (non représentée) du conduit 8 est reliée à un dispositif de gonflage (également non représenté) susceptible d'adresser un fluide de gonflage à l'intérieur du manchon 6, par exemple du gaz ou un liquide à une pression de 2 ou 3 bars, par l'intermédiaire dudit conduit 8 et du raccord souple 10. Ainsi, le fluide pénétrant dans le manchon peut en sortir par le raccord souple 11 et le conduit 9, de façon à ce que ledit fluide balaye le manchon. En contrôlant le débit dudit fluide à l'extrémité proximale (non representée) du conduit 9, on peut régler la pression à l'intérieur dudit manchon.

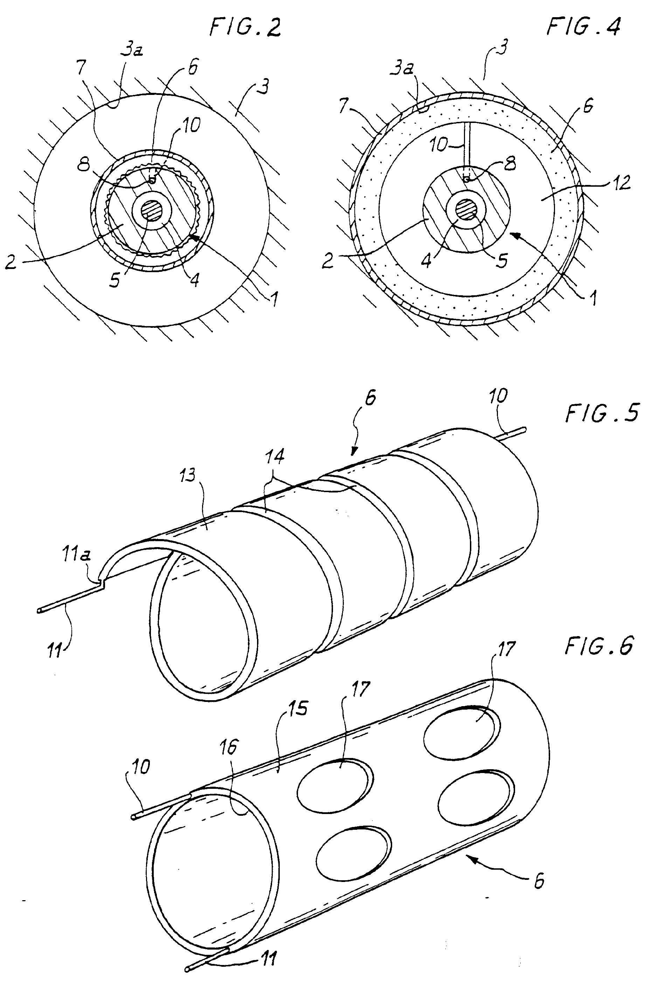

Le manchon 6 étant à l'état dégonflé et comprimé par la membrane élastique 7 (figures 1 et 2), le cathéter 1 est introduit dans le conduit morphologique 3, grâce au support de guidage 5 et mis en place de façon précise grâce à des repères radio-opaques (non représentés).

Lorsque le manchon 6 est ainsi amené à l'emplacement désiré du conduit morphologique 3, du fluide de gonflage est adressé au manchon 6, de la façon indiquée ci-dessus. Le manchon 6 se dilate alors jusqu'à ce que sa paroi externe recouverte de la membrane 7 soit plaquée contre la paroi interne 3a du conduit morphologique 3, un espace annulaire 12 étant alors ménagé entre ledit manchon 6 et l'extrémité distale 2 du cathéter 1 (voir les figures 3 et 4).

Dans cette position gonflée, le manchon 6 reste relié au conduits 8 et 9, grâce aux raccords souples 10 et 11, qui traversent ledit espace 12.

L'expérience a montré que, pour obtenir une bonne adhérence du manchon 6 sur la paroi interne 3a du conduit morphologique 3, il était suffisant que la pression du fluide de gonflage soit de 2 à 3 bars, pression qui est tolérable par le patient. De plus, il est avantageux à cette fin que le diamètre D du conduit morphologique 3 soit inférieur, par exemple d'environ 10 %, au diamètre externe que prendrait le manchon 6 à sa pression d'utilisation à l'intérieur dudit conduit 3 gonflé, s'il n'était pas enfermé dans le conduit morphologique 3.

Dans le cas où le manchon 6 doit constituer une prothèse provisoire, la simple circulation du fluide à travers le manchon 6 peut entretenir la rigidité de celui-ci à l'état gonflé.

En revanche, lorsque le manchon 6 doit constituer une prothèse permanente, le fluide de gonflage peut être une résine (acrylique, époxyde) ou un latex polymérisable, de préférence à température ambiante. Après polymérisation dudit fluide, le manchon garde sa forme rigide gonflée.

Il est alors préférable de prévoir sur les raccords souples 10 et 11 des zones fragilisées 10a et 11a, respectivement, permettant de rompre lesdits raccords, par exemple par torsion et/ou par traction du cathéter 1. Après rupture desdites zones fragilisées 10a et 11a, le cathéter 1 peut être retiré du conduit morphologique 3, seul le manchon rigide gonflé 6 restant en place.

La polymérisation du manchon 6 à l'état gonflé peut également être obtenue en introduisant dans le manchon 6 une matière qui, par réaction avec le fluide de gonflage, est susceptible de rigidifier le manchon gonflé.

Bien entendu, quel que soit le mode de rigidification du manchon gonflé, la solidification doit intervenir dans un temps court pour minimiser la durée de l'intervention.

Dans le cas d'une prothèse permanente à manchon rigidifiable, le cathéter 1 sera du type jetable, car le fluide de gonflage sera également solidifié dans les conduits 8 et 9.

On remarquera que, dans certains cas, le support de guidage 5 pourra être remplacé, en cours d'intervention, par une fibre optique capable de transmettre l'énergie d'un rayonnement ou par un tube revêtu d'un revêtement intérieur permettant d'assurer la transmission d'ultra-sons depuis une source extérieure jusqu'à la partie à traiter.

Le manchon gonflable 6 peut être réalisé de différentes façons. De préférence, il est réalisé en une matière préformable, telle qu'un polyéthylène irradié. Ainsi, sa forme à l'état gonflé peut être adaptée à celle de l'emplacement du conduit 3, où il est destiné à former prothèse.

Dans le mode de réalisation illustré par la figure 5, le manchon 6 est constitué par un tube plat préformé 13 enroulé en serpentin hélicoïdal. On remarquera que ce mode de réalisation est avantageux car, entre les spires non jointives du serpentin, le manchon 6 présente des espaces 14 à travers lesquels peut circuler le sang. Ainsi, si le manchon est disposé dans un conduit 3, à l'intersection avec un ou plusieurs autres conduits morphologiques, le sang peut continuer à circuler entre ces conduits, à travers les espaces 14. A cet effet, il est nécessaire que la membrane 7 soit elle-même ajourée ou, mieux, présente la forme d'un filet. Bien entendu, le manchon peut être constitué de plusieurs serpentins hélicoïdaux à spires emboîtées, avec entrées et sorties de gonflage indépendantes. Ces serpentins présentent éventuellement des diamètres différents et des communications internes et externes peuvent être envisagées entre lesdits serpentins.

Sur la figure 6, le manchon 6 est formé par deux parois 15 et 16 soudées, des lumières 17 étant ménagées dans la paroi latérale dudit manchon, pour assurer la circulation sanguine périphérique à travers la double paroi dudit manchon.

Bien entendu, quelle que soit sa réalisation, le manchon 6 est en une matière biocompatible.

## Revendications

1. Cathéter (1) pour conduit morphologique (3), comportant un ballon gonflable (6), disposé à l'extrémité distale (2) dudit cathéter et relié à l'extrémité proximale dudit cathéter par au moins un conduit de gonflage (8) susceptible d'être relié à un dispositif de gonflage, ledit ballon gonflable (6) pouvant prendre, soit un état dégonflé pour lequel il est plaqué contre ladite extrémité distale dudit cathéter, soit un état gonflé pour lequel il est plaqué par sa paroi externe contre la paroi interne (3a) dudit conduit morphologique (3),
caractérisé en ce que ledit ballon gonflable (6) présente la forme d'un manchon entourant ladite extrémité distale (2) dudit cathéter et relié à celle-ci par un raccord souple (10), lui-même relié audit conduit de gonflage (8) et en ce que, à l'état gonflé, ledit manchon ménage un espace annulaire (12) entre sa paroi interne et ladite extrémité distale dudit cathéter.

2. Cathéter selon la revendication 1,
caractérisé en ce qu'il comporte une membrane élastique (7) entourant ledit manchon (6) et le comprimant contre ladite extrémité distale (2) du cathéter.

3. Cathéter selon la revendication 2,
caractérisé en ce que ladite membrane élastique (7) est ajourée.

4. Cathéter selon l'une quelconque des revendications 1 à 3,
caractérisé en ce que ledit dispositif de gonflage adresse un fluide de gonflage susceptible de rigidifier ledit manchon dans son état gonflé et en ce que ledit raccord souple est susceptible d'être rompu (en 10a).

5. Cathéter selon l'une quelconque des revendications 1 à 4,
caractérisé en ce qu'il comporte de plus un conduit de vidange (9) reliant ledit manchon (6) à l'extrémité proximale du cathéter, par l'intermédiaire d'un raccord souple (11) reliant ledit manchon à ladite extrémité distale dudit cathéter.

6. Cathéter selon la revendication 5, dans lequel ledit fluide de gonflage est susceptible de rigidifier ledit manchon dans son état gonflé,
caractérisé en ce que ledit raccord souple (11) est susceptible d'être rompu (en 11a).

7. Cathéter selon l'une des revendications 1 à 6,
caractérisé en ce que ledit manchon (6) est formé par un tube (13) enroulé en serpentin hélicoïdal.

8. Cathéter selon la revendication 7,
caractérisé en ce que les spires dudit serpentin ne sont pas jointives.

9. Cathéter selon l'une des revendications 1 à 6,
caractérisé en ce que ledit manchon (6) est un tube à double paroi (15, 16).

10. Cathéter selon la revendication 9,
caractérisé en ce que ledit tube à double paroi est percé d'ouvertures latérales (17).

11. Cathéter selon l'une des revendications 1 à 10,
caractérisé en ce que ledit manchon est préformé.

12. Cathéter selon la revendication 11,
caractérisé en ce que le diamètre extérieur dudit manchon, gonflé hors dudit conduit morphologique (3) à la pression d'utilisation à l'intérieur de celui-ci, est supérieur d'environ 10 % au diamètre dudit conduit morphologique.

## Patentansprüche

1. Katheder (1) für eine Leitung (3) eines Organismus, die einen aufblähbaren Ballon (6) umfaßt, der am distalen Ende (2) des Katheders angeordnet ist und mit dem proximalen Ende des Katheders durch wenigstens eine Leitung zum Aufblähen (8) verbunden ist, die dafür geeignet ist, mit einer Aufblähvorrichtung verbunden zu werden, wobei der aufblähbare Ballon (6) entweder einen leeren Zustand, in dem er am distalen Ende des Katheders anliegt, oder einen aufgeblähten Zustand, in dem er mit seiner äußeren Seitenwand an der inneren Seitenwand (3a) der Organismusleitung (3) anliegt, einnehmen kann,
dadurch gekennzeichnet, daß der aufblähbare Ballon (6) die Form einer Hülse aufweist, die das distale Ende (2) des Katheders umschließt und mit diesem über eine dehnbare Verbindung (10) verbunden ist, die ihrerseits mit der Aufblähleitung (8) verbunden ist und dadurch gekennzeichnet, daß in aufgeblähtem Zustand die Hülse einen ringförmigen Zwischenraum (12) zwischen ihrer inneren Seitenwand und dem distalen Ende des Katheders ausbildet.

2. Katheder nach Anspruch 1, dadurch gekennzeichnet, daß er eine elastische Membran (7) umfaßt, die die Hülse (6) umschließt und diese gegen das distale Ende (2) des Katheders zusammendrückt.

3. Katheder nach Anspruch 2, dadurch gekennzeichnet, daß die elastische Membran (7) gelocht ist.

4. Katheder nach einem der Ansprüche 1 bis 3, dadurch gekennzeichnet, daß die Aufblähvorrichtung ein Aufblähfluid zuführt, das in der Lage ist, die Hülse in ihrem aufgeblähten Zustand zu versteifen und dadurch, daß die dehnbare Verbindung dafür ausgebildet ist, abgebrochen zu werden (siehe 10a).

5. Katheder nach einem der Ansprüche 1 bis 4, dadurch gekennzeichnet, daß er außerdem eine Entleerungsleitung (9) umfaßt, die die Hülse über eine die Hülse mit dem distalen Ende des Katheders verbindende dehnbare Verbindung (11) mit dem proximalen Ende des Katheders verbindet.

6. Katheder nach Anspruch 5, in dem das Aufblähfluid dazu geeignet ist, die Hülse in aufgeblähtem Zustand zu versteifen, dadurch gekennzeichnet, daß die dehnbare Verbindung (11) dafür ausgebildet ist, abgebrochen zu werden (siehe 11a).

7. Katheder nach einem der Ansprüche 1 bis 6, dadurch gekennzeichnet, daß die Hülse (6) durch eine Röhre (13) gebildet wird, die spiralförmig aufgerollt ist.

8. Katheder nach Anspruch 7, dadurch gekennzeichnet, daß die Windungen der Spirale nicht fugendicht miteinander verbunden sind.

9. Katheder nach einem der Ansprüche 1 bis 6, dadurch gekennzeichnet, daß die Hülse (6) eine Röhre mit doppelter Wand (15, 16) ist.

10. Katheder nach Anspruch 9, dadurch gekennzeichnet, daß die doppelwandige Röhre von seitlichen Öffnungen (17) durchsetzt ist.

11. Katheder nach einem der Ansprüche 1 bis 10, dadurch gekennzeichnet, daß die Hülse vorgeformt ist.

12. Katheder nach Anspruch 11, dadurch gekennzeichnet, daß der äußere Durchmesser der Hülse, wenn sie außerhalb der Organismusleitung (3) unter dem Druck, der während der Benutzung in deren Inneren herrscht, aufgeblasen ist, um ungefähr 10% größer ist als der Durchmesser der Organismusleitung.

## Claims

1. Catheter (1) for a morphological channel (3), the catheter (1) comprising an inflatable balloon (6), arranged at the distal end (2) of the said catheter and connected to the proximal end of the said catheter via at least one inflation conduit (8) capable of being connected to an inflating device, it being possible for the said inflatable balloon (6) to assume either a deflated state, in which it is flattened against the said distal end of the said catheter, or an inflated state, in which it is flattened via its outer wall against the inner wall (3a) of the said morphological channel (3), characterized in that the said inflatable balloon (6) is in the form of a sleeve surrounding the said distal end (2) of the said catheter and connected to the latter via a flexible joining piece (10), itself connected to the said inflation conduit (8), and in that, in the inflated state, the said sleeve leaves an annular space (12) between its inner wall and the said distal end of the said catheter.

2. Catheter according to Claim 1, characterized in that it comprises an elastic membrane (7) surrounding the said sleeve (6) and compressing it against the said distal end (3) of the catheter.

3. Catheter according to Claim 2, characterized in that the said elastic membrane (7) is open-worked.

4. Catheter according to any one of Claims 1 to 3, characterized in that the said inflating device delivers an inflation fluid capable of stiffening the said sleeve in its inflated state, and in that the said flexible joining piece is capable of being broken (at 10a).

5. Catheter according to any one of Claims 1 to 4, characterized in that it additionally comprises an emptying conduit (9) connecting the said sleeve (6) to the proximal end of the catheter, by way of a flexible joining piece (11) connecting the said sleeve to the said distal end of the said catheter.

6. Catheter according to Claim 5, in which the said inflation fluid is capable of stiffening the said sleeve in its inflated state, characterized in that the said flexible joining piece (11) is capable of being broken (at 11a).

7. Catheter according to one of Claims 1 to 6, characterized in that the said sleeve (6) is formed by a tube (13) wound as a serpentine pipe.

8. Catheter according to Claim 7, characterized in that the turns of the said serpentine pipe are not contiguous.

9. Catheter according to one of Claims 1 to 6, characterized in that the said sleeve (6) is a tube with a double wall (15, 16).

10. Catheter according to Claim 9, characterized in that the said tube with a double wall is pierced with lateral openings (17).

11. Catheter according to one of Claims 1 to 10, characterized in that the said sleeve is preformed.

12. Catheter according to Claim 11, characterized in that the external diameter of the said sleeve, inflated outside the said morphological channel (3) at the pressure of use inside the latter, is greater by approximately 10 % than the diameter of the said morphological channel.
